# EUROPEAN PATENT APPLICATION

(11) **EP 4 428 245 A1**
(43) Date of publication of application: **11.09.2024**
(21) Application number: 22889922.5
(22) Date of filing: 31.10.2022
(51) Int. Cl.: C12Q 1/682, G01N 33/53, G01N 33/536

(54) **METHOD FOR DETECTING AMYLOID-BETA BOUND TO BRAIN-NEURONAL-CELL-DERIVED EXOSOME IN BLOOD, KIT FOR DETECTION OF AMYLOID-BETA BOUND TO BRAIN-NEURONAL-CELL-DERIVED EXOSOME IN BLOOD, AND METHOD FOR EVALUATING ACCUMULATION LEVEL OF AMYLOID-BETA IN BRAIN**

(30) Priority: 04.11.2021 JP 2021180612
(71) Applicant: Toppan Holdings Inc., Tokyo 110-0016 (JP); National University Corporation Hokkaido University, Sapporo-shi, Hokkaido 060-0808 (JP)
(72) Inventor: MAKINO, Yoichi, Tokyo 110-0016 (JP); HIRASE, Takumi, Tokyo 110-0016 (JP); YUYAMA, Kohei, Sapporo-shi, Hokkaido 060-0808 (JP)
(74) Representative: TBK
(86) International application number: PCT/JP2022/040614
(87) International publication number: WO 2023/080102

(57) **Abstract**

There are provided a method for detecting amyloid-β bound to a brain-neuronal-cell-derived exosome in blood, the method including: a step of binding amyloid-β bound to the brain-neuronal-cell-derived exosome in blood to an amyloid-β-specific binding substance that specifically binds to amyloid-β, labeled with a nucleic acid fragment; and a step of detecting the amyloid-β-specific binding substance, and a method for evaluating an accumulation level of amyloid-β in a brain, the method including: a step of detecting amyloid-β bound to the brain-neuronal-cell-derived exosome in blood; and a step of evaluating an accumulation level of amyloid-β in a brain based on the detected amount of the amyloid-β.

## Description

### TECHNICAL FIELD

The present invention relates to a method for detecting amyloid-β bound to a brain-neuronal-cell-derived exosome in blood, a kit for detection of amyloid-β bound to a brain-neuronal-cell-derived exosome in blood, and a method for evaluating an accumulation level of amyloid-β in a brain.

Priority is claimed on Japanese Patent Application No. 2021-180612, filed November 4, 2021, the content of which is incorporated herein by reference.

### BACKGROUND ART

Alzheimer's disease is the form of dementia with the largest number of patients, with Alzheimer's disease occupying a proportion of 50% to 75% of all forms of dementia, and treatment methods for it are being developed.

One cause of Alzheimer's disease is considered to be amyloid-β forming a polymer outside a brain-neuronal-cell, accumulating in the brain, and depositing in the brain to form senile plaques, and subsequently, in the brain-neuronal-cell, a protein called tau is hyperphosphorylated, forms a filament, deposits in the brain to form neurofibrils, and causes neurological disorders such as neurocyte death and synaptic dysfunction, resulting in degeneration of the neurons.

It is effective to perform treatment or prevention of Alzheimer's disease during the accumulation of amyloid-β before the tau-dependent neurological disorders in the brain are caused. For that purpose, a method that enables the amyloid-β in the brain to be measured is required, but a cerebrospinal fluid examination, which is currently used as a method for measuring amyloid-β, is highly invasive, and a PET imaging method or a method for detecting amyloid-β in blood by mass spectrometry requires special equipment.

An exosome is a type of extracellular vesicle, and is a membrane vesicle having a diameter of about 40 to 150 nm secreted from a cell. In recent years, it has been pointed out that there is a possibility that an exosome plays a role in transmitting information to distant cells or tissues. An exosome contains various proteins, lipids, and nucleic acids, which are considered to cause functional or physiological changes by being transported to other cells. Non-Patent Document 1 reports that amyloid-β binds to ganglioside GM1 present on a surface of an exosome of a neuronal cell, and amyloid-β is transported by the exosome and accumulates in the brain to form plaques. In addition, Non-Patent Document 2 reports that amyloid-β is detected in an exosome isolated from plasma of an Alzheimer's disease patient.

On the other hand, early discovery of diseases and prediction of effects of administration have been carried out by quantitatively detecting target molecules in biological samples. The quantification of nucleic acids such as DNA and RNA is carried out by a real-time PCR method and the like.

In recent years, there has been an increasing need for more accurate detection of target molecules for the purpose of early discovery of diseases and the like. As a technique of accurately detecting a target molecule, for example, Non-Patent Document 3 discloses a technology in which an enzymatic reaction is carried out in a large number of micro-compartments to detect fluorescent signals. This technique is referred to as digital measurement.

In digital measurement, a sample solution is divided into a very large number of minute solutions. Then, a signal from each minute solution is binarized, only the presence or absence of the target molecule is discriminated, and the number of target molecules is measured. According to the digital measurement, compared to the real-time PCR and the like in the related art, it is possible to significantly improve detection sensitivity and quantitative properties.

In digital PCR, which is one type of digital measurement, a mixture of a PCR reaction reagent and a nucleic acid is diluted such that the number of nucleic acids serving as a template present in one micro-droplet in one micro-compartment is 0 or 1. In the digital PCR, it is preferable that a volume of each micro-droplet be small in order to increase the sensitivity of nucleic acid amplification and to simultaneously perform nucleic acid amplification for a large number of micro-droplets. For example, Non-Patent Document 3 discloses a method using micro-sized liquid droplets formed such that the volume of each well is several nanoliters.

In addition, as another technique of digital measurement, there is digital invasive cleavage assay (ICA). Patent Document 1 discloses a technique of amplifying a DNA sample by a PCR reaction, introducing a denatured PCR product into a device having microwells, and during detection, detecting DNA by an Invader (registered trademark) method without amplifying the DNA.

### Citation List

### Patent Document

Patent Document 1: PCT International Publication No. WO2015/115635

### Non-Patent Document

Non-Patent Document 1: Lawrence Rajendran et al., Alzheimer's disease β-amyloid peptides are released in association with exosomes. Proc. Natl. Acad. Sci. USA 2006 July 25; 103 (30): 11172-11177.
Non-Patent Document 2: Massimo S. Fiandaca et ai., Identification of preclinical Alzheimer's disease by a profile of pathogenic proteins in neurally derived blood exosomes: A case-control study. Alzheimer's & Dementia 11 (2015) 600-607
Non-Patent Document 3: Olmedillas-Lopez S., et al., Current and Emerging Applications of Droplet Digital PCR in Oncology. Mol Diagn Ther. 2017 Oct; 21 (5): 493-510

### SUMMARY OF INVENTION

### Technical Problem

In Non-Patent Document 1, amyloid-β bound to an exosome released from a cultured neuronal cell is detected; however, this detection method is a method of isolating an exosome released from a cultured neuronal cell, immunostaining amyloid-β bound to the exosome, and detecting the amyloid-β using an electron microscope, and is not a method of rapidly detecting amyloid-β bound to a brain-neuronal-cell-derived exosome in blood. In even Non-Patent Document 2, amyloid-β in an exosome isolated from plasma is detected, and is not a rapid detection method. In addition, it is not known how to evaluate an accumulation level of amyloid-β in the brain by detecting the amyloid-β bound to an exosome in blood.

The present Invention has been made in view of the above circumstances, and an object of the present invention is to provide a technology of detecting amyloid-β bound to a brain-neuronal-cell-derived exosome rapidly and with high sensitivity and a technology of evaluating an accumulation level of amyloid-β in the brain.

### Solution to Problem

The present invention includes the following aspects.
[1] A method for detecting amyloid-β bound to a brain-neuronal-cell-derived exosome in blood, the method including: a step of binding amyloid-β bound to the brain-neuronal-cell-derived exosome in blood to an amyloid-β-specific binding substance that specifically binds to amyloid-β, labeled with a nucleic acid fragment; and a step of detecting the amyloid-β-specific binding substance.
[2] The detection method according to [1], further including: a step of detecting a biomarker present on a surface of the brain-neuronal-cell-derived exosome.
[3] A method for detecting amyloid-β bound to a brain-neuronal-cell-derived exosome in blood, the method including: a step of binding a biomarker present on a surface of the exosome to a biomarker-specific binding substance on the surface of the exosome, which specifically binds to the biomarker present on the surface of the exosome, labeled with a nucleic acid fragment; a step of detecting amyloid-β bound to the brain-neuronal-cell-derived exosome in blood; and a step of detecting the biomarker-specific binding substance on the surface of the exosome.
[4] The detection method according to any one of [1] to [3], in which the amyloid-β is bound to a biomarker present on a surface of the brain-neuronal-cell-derived exosome.
[5] The detection method according to any one of [2] to [4], in which the step of detecting the biomarker or the amyloid-β is carried out by capturing the brain-neuronal-cell-derived exosome using a substance that specifically binds to the biomarker or the amyloid-β.
[6] The detection method according to any one of [1] to [5], in which the amyloid-β-specific binding substance is an antibody or antibody fragment that specifically binds to amyloid-β.
[7] The detection method according to any one of [2] to [6], in which the biomarker is ganglioside GM1.
[8] The detection method according to any one of [1] to [7], in which the step of detecting the amyloid-β-specific binding substance is carried out by an invasive cleavage assay.
[9] A kit for detection of amyloid-β bound to a brain-neuronal-cell-derived exosome in blood, the kit including: an amyloid-β-specific binding substance that specifically binds to amyloid-β, labeled with a nucleic acid fragment.
[10] The kit according to [9], further including: a substance that detects a biomarker present on a surface of the brain-neuronal-cell-derived exosome.
[11] The kit according to [10], in which the substance that detects a biomarker present on the surface of the brain-neuronal-cell-derived exosome is a substance that specifically binds to the biomarker present on the surface of the brain-neuronal-cell-derived exosome.
[12] A kit for detection of amyloid-β bound to a brain-neuronal-cell-derived exosome in blood, including: a biomarker-specific binding substance on a surface of a brain-neuronal-cell-derived exosome, which specifically binds to a biomarker present on the surface of the exosome, labeled with a nucleic acid fragment; and an amyloid-β-specific binding substance that specifically binds to amyloid-β.
[13] The kit according to any one of [9] to [12], in which the amyloid-β-specific binding substance is an antibody or antibody fragment that specifically binds to amyloid-β.
[14] The kit according to any one of [10] to [13], in which the biomarker is ganglioside GM1.
[15] A method for evaluating an accumulation level of amyloid-β in a brain, the method including: a step of detecting amyloid-β bound to a brain-neuronal-cell-derived exosome in blood; and a step of evaluating an accumulation level of amyloid-β in a brain based on the detected amount of the amyloid-β.
[16] The evaluation method according to [15], in which detection of the amyloid-β bound to the brain-neuronal-cell-derived exosome is carried out by the detection method according to any one of [1] to [8].

### Advantageous Effects of Invention

According to the present invention, it is possible to provide a technology of detecting amyloid-β bound to a brain-neuronal-cell-derived exosome rapidly and with high sensitivity and a technology of evaluating an accumulation level of amyloid-β in the brain.

### BRIEF DESCRIPTION OF DRAWINGS

FIG. 1 is a schematic diagram showing a method for detecting amyloid-β bound to a brain-neuronal-cell-derived exosome of a first embodiment.
FIG. 2 is a schematic cross-sectional diagram showing an example of a fluid device.
FIG. 3 is a schematic cross-sectional diagram showing an example of a fluid device.
FIG. 4 is a schematic cross-sectional diagram showing an example of a fluid device.
FIG. 5 is a schematic cross-sectional diagram showing an example of a fluid device.
FIG. 6 is a schematic cross-sectional diagram showing an example of a fluid device.
FIG. 7 is a schematic cross-sectional diagram showing an example of a fluid device.
FIG. 8 is a schematic diagram showing an example of an invasive cleavage assay (ICA) method.
FIG. 9 is a schematic diagram showing a method for detecting amyloid-β bound to a brain-neuronal-cell-derived exosome of a second embodiment.
FIG. 10 is a diagram showing a fluorescence image in Experimental Example 1.
FIG. 11 is a graph showing fluorescence intensity in Experimental Example 1. The left figure shows the fluorescence intensity of the linear scale, and the right figure shows the fluorescence intensity of the log scale.
FIG. 12 is a graph showing fluorescence intensity of an exosome of a 3-week-old or 13-week-old human familial mutant APP gene-introduced mouse in Experimental Example 2. "Control" shows a case where a buffer solution (PBS) was used instead of an exosome.
FIG. 13 is a graph showing concentrations of amyloid-β40 (Aβ40) and amyloid-β42 (Aβ42) in a brain tissue of a human familial mutant APP gene-introduced mouse in Experimental Example 3. The left figure shows a concentration of amyloid-β40, and the right figure shows a concentration of amyloid-β42.
FIG. 14 is a diagram showing immunohistochemical staining of amyloid-β in a brain tissue of a 3-week-old or 13-week-old human familial mutant APP gene-introduced mouse in Experimental Example 3. The left figure shows immunohistochemical staining of amyloid-β in the brain tissue of the 3-week-old human familial mutant APP gene-introduced mouse, and the right figure shows immunohistochemical staining of amyloid-β in the brain tissue of the 13-week-old human familial mutant APP gene-introduced mouse.
FIG. 15 is a graph showing a concentration of free amyloid-β40 (Aβ40) or amyloid-β42 (Aβ42) in a serum of a 3-week-old or 13-week-old human familial mutant APP gene-introduced mouse in Comparative Example 1. The left figure shows a concentration of amyloid-β40, and the right figure shows a concentration of amyloid-β42.

### DESCRIPTION OF EMBODIMENTS

Hereinafter, embodiments of the present invention will be described in detail with reference to the drawings depending on the case. In the drawings, the same or corresponding parts are denoted by the same or corresponding reference numerals, and overlapping descriptions are omitted. Note that dimensional ratios in each drawing are exaggerated for the sake of explanation, and do not necessarily match the actual dimensional ratios.

### [Method for detecting amyloid-β]

### <First embodiment>

The present invention provides, in one embodiment, a method for detecting amyloid-β bound to a brain-neuronal-cell-derived exosome in blood, the detection method including a step of binding amyloid-β bound to the brain-neuronal-cell-derived exosome in blood to an amyloid-β-specific binding substance that specifically binds to amyloid-β, labeled with a nucleic acid fragment; and a step of detecting the amyloid-β-specific binding substance. Hereinafter, a detection method according to the present embodiment will be described.

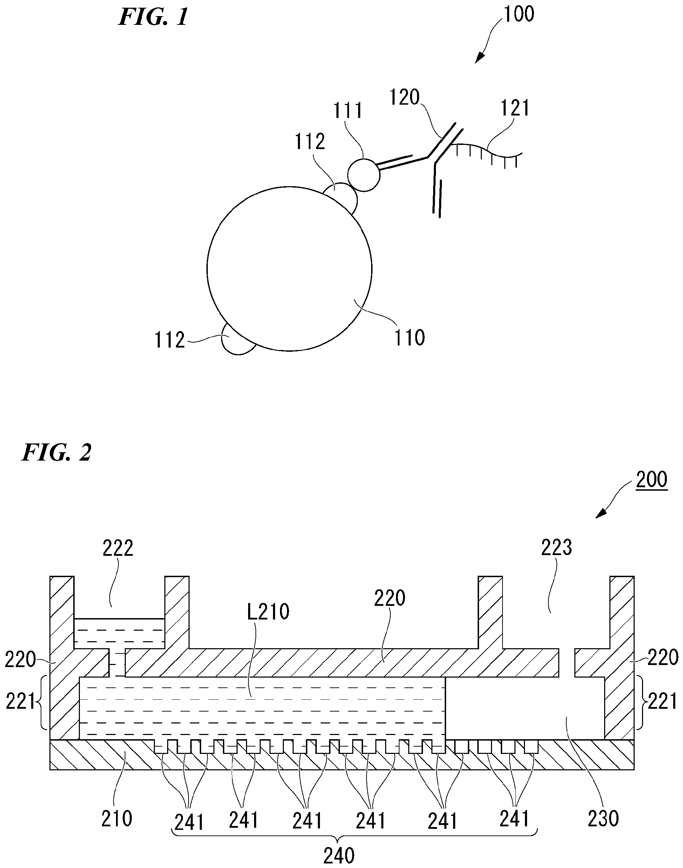

FIG. 1 is a schematic diagram showing a detection method of the present embodiment. As shown in FIG. 1, in the detection method of the present embodiment, first, amyloid-β 111 bound to a brain-neuronal-cell-derived exosome 110 in blood and an amyloid-β-specific binding substance 120 that specifically binds to the amyloid-β 111, labeled with a nucleic acid fragment 121, are bound to each other. The amyloid-β 111 binds to a biomarker 112 present on a surface of the brain-neuronal-cell-derived exosome 110. Examples of blood include whole blood, serum, plasma, and the like.

Exosomes are membrane vesicles that are secreted from most cells, and have a diameter of about 40 nm to 150 nm.

In a living body, exosomes are observed in bodily fluids such as saliva, blood, urine, amniotic fluid, and malignant ascites, and are also secreted from cultured cells. In recent years, it has been pointed out that there is a possibility that an exosome plays a role in transmitting information to distant cells or tissues. An exosome contains various proteins, lipids, and nucleic acids, which are considered to cause functional or physiological changes by being transported to other cells. Specific examples thereof have been suggested to have roles such as mediation of adaptive immune responses against infectious agents and tumors, tissue repair, neurotransmission, and transport of pathogenic proteins.

In the detection method of the present embodiment, the exosome is a brain-neuronal-cell-derived exosome. Amyloid-β, which is considered to be a causative factor of Alzheimer's disease, is known to be transported by binding to a brain-neuronal-cell-derived exosome, and to accumulate in the brain of an Alzheimer's disease patient to form plaques. Therefore, it is possible to detect amyloid-β bound to a brain-neuronal-cell-derived exosome by using a substance that specifically binds to the amyloid-β bound to the brain-neuronal-cell-derived exosome.

Amyloid-β 111 binds to the exosome 110 by binding to the biomarker 112 present on a membrane surface of the brain-neuronal-cell-derived exosome 110. Examples of the biomarker 112 present on the membrane surface of the brain-neuronal-cell-derived exosome 110, to which amyloid-β 111 can be bound, include ganglioside GM1 and the like.

Subsequently, an amyloid-β-specific binding substance 120, labeled with the nucleic acid fragment 121, bound to the amyloid-β 111 bound to the brain-neuronal-cell-derived exosome 110, is detected. In a case where the detection method of the present embodiment is suitable for detection by digital measurement, it is preferable to use a fluid device having a well array in which a plurality of wells is arranged.

### (Fluid device)

FIG. 2 is a schematic cross-sectional diagram showing an example of a fluid device. As shown in FIG. 2, the fluid device 200 includes a substrate 210 and a lid member 220 arranged to face the substrate 210. The lid member 220 has a convex portion 221, and a tip of the convex portion 221 is in contact with the substrate 210. In the fluid device 200, a well array 240 is integrally molded with the substrate 210 on one surface of the substrate 210 and faces the lid member 220. The well array 240 has a plurality of wells 241. The lid member 220 may be welded or bonded to the substrate 210.

The well 241 is open on the surface of the substrate 210. The shape, dimension, and arrangement of the wells 241 are not particularly limited, but one brain-neuronal-cell-derived exosome is preferably introduced into one well 241. The well 241 is preferably a micro well having a small volume. For example, the volume of one well 241 may be about 10 fL to 100 pL. In the fluid device 200, the plurality of the wells 241 of the same shape and the same size constitutes a well array 240. The same shape and the same size may be the same shape and the same capacity to an extent required for carrying out digital measurement, and variations to an extent of manufacturing error are allowed.

A diameter of the well 241 may be, for example, about 1 µm to 10 µm, and a depth of the well 241 may be, for example, about 1 µm to 10 µm. In addition, the arrangement of the wells 241 is not particularly limited, and for example, the wells 241 may be arranged in a triangular lattice, may be arranged in a square lattice, or may be randomly arranged.

In the fluid device 200, a space is formed between the well array 240 and the lid member 220 due to the presence of the convex portion 221. The space constitutes a flow path 230. The flow path 230 functions as a path for feeding a liquid in which a brain-neuronal-cell-derived exosome, an amyloid-β-specific binding substance, labeled with a nucleic acid fragment, and the like are dispersed, and a sealing liquid that will be described later. The shape, structure, capacity, and the like of the flow path 230 are not particularly limited, but a height of the flow path 230 (the distance between the surface of the substrate 210 and the surface of the lid member 220 facing the substrate 210) is, for example, 500 µm or less, may be, for example, 300 µm or less, may be, for example, 200 µm or less, or may be, for example, 100 µm or less.

The convex portion 221 may be integrally molded with the lid member 220. The lid member 220 can be molded into a plate shape having the convex portion 221 by, for example, molding a fluid body of a thermoplastic resin using a molding die. In addition, a reagent introduction port 222 and a reagent discharge port 223 may be formed in the lid member 220.

In a case where the lid member 220 has the convex portion 221, the lid member 220 and the substrate 210 are stacked so that the convex portion 221 is in contact with a surface of the substrate 210 on which the well 241 is open. As a result, a space between the lid member 220 and the substrate 210 serves as the flow path 230. The lid member 220 and the substrate 210 may be welded by laser welding or the like.

### (Modification example 1 of fluid device)

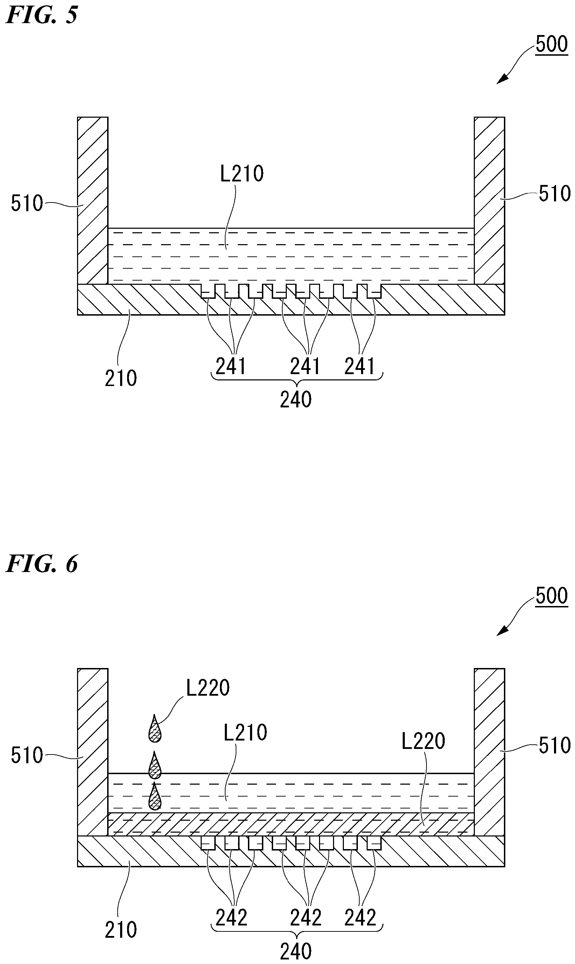

The fluid device used in the detection method of the present embodiment is not limited to the above-described fluid device 200. FIG. 5 is a schematic cross-sectional diagram showing an example of the fluid device. As shown in FIG. 5, a fluid device 500 includes the substrate 210 and a wall member 510. In the fluid device 500, the well array 240 is integrally molded with the substrate 210 on one surface of the substrate 210. The well array 240 has a plurality of wells 241.

The fluid device 500 is mainly different from the above-described fluid device 200 in that the fluid device 500 does not include the lid member 220. Therefore, the fluid device 500 does not have a flow path.

### (Modification example 2 of fluid device)

In the above-described fluid device 200, the lid member 220 and the convex portion 221 are integrally molded. However, the lid member 220 and the convex portion 221 may be molded as separate bodies.

In addition, in the above-described fluid device 200 and fluid device 500, the well array 240 is integrally molded with the substrate 210 on one surface of the substrate 210. However, the well array may not be integrally molded with the substrate 210. For example, the well array 240 molded as a separate body from the fluid device may be arranged on the substrate 210 of the fluid device. Alternatively, a resin layer may be laminated on the surface of the substrate 210, and a well array may be formed on the resin layer by etching or the like.

### (Material of fluid device)

The substrate 210 is formed using, for example, a resin. A type of the resin is not particularly limited, but a resin that is resistant to reagents and sealing liquids is preferable. In addition, in a case where a signal to be detected is fluorescence, a resin with less autofluorescence is preferable. Examples of the resin include a cycloolefin polymer, a cycloolefin copolymer, silicone, polypropylene, polycarbonate, polystyrene, polyethylene, polyvinyl acetate, a fluororesin, an amorphous fluororesin, and the like, but are not limited thereto.

A plurality of the wells 241 may be formed on one surface of the substrate 210 in a plate thickness direction. Examples of a method of forming a well using a resin include injection molding, thermal imprint, optical imprint, and the like.

Alternatively, for example, a fluororesin may be laminated on the substrate 210, and the fluororesin may be processed by etching or the like to form a well array. As the fluororesin, for example, CYTOP (registered trademark) (AGC Inc.) or the like can be used.

In addition, in a case where the fluid device has the lid member 220, the material of the lid member 220 is preferably a resin with less autofluorescence, and may be, for example, a thermoplastic resin such as a cycloolefin polymer and a cycloolefin copolymer.

In addition, the lid member 220 may be formed of a material that does not transmit light having a wavelength in the vicinity of a wavelength detected when the signal is observed in fluorescence, or may be formed of a material that does not transmit light at all. For example, the lid member 220 may be formed of a thermoplastic resin to which carbon, metal particles, or the like is added.

### (Detection method of present embodiment)

Next, the detection method of the present embodiment will be described by taking a case where the fluid device 200 is used as an example while referring to FIGS. 2 to 4 depending on the case. The detection method of the present embodiment is a detection method of amyloid-β 111 bound to a brain-neuronal-cell-derived exosome 110 in blood, the method including a step of binding a brain-neuronal-cell-derived exosome

110 in blood to an amyloid-β-specific binding substance 120 that specifically binds to the amyloid-β, labeled with a nucleic acid fragment 121; and a step of detecting the amyloid-β-specific binding substance 120. According to the method of the present embodiment, it is possible to detect amyloid-β 111 bound to a brain-neuronal-cell-derived exosome 110.

The detection method of the present embodiment may further include a step of detecting a biomarker 112 present on a surface of a brain-neuronal-cell-derived exosome 110. The step of detecting the biomarker 112 present on the surface of the brain-neuronal-cell-derived exosome 110 can be carried out by detecting the biomarker 112 present on the surface of the brain-neuronal-cell-derived exosome 110 using a substance that detects the biomarker 112 present on the surface of the brain-neuronal-cell-derived exosome 110. In a case where the biomarker 112 is present on the surface of the brain-neuronal-cell-derived exosome 110, the brain-neuronal-cell-derived exosome 110 can be captured using a substance (a biomarker-specific binding substance on the surface of the exosome) that specifically binds to the biomarker 112 present on the surface of the brain-neuronal-cell-derived exosome 110, and thus detection of the biomarker 112 present on the surface of the brain-neuronal-cell-derived exosome 110 can be carried out by capturing the brain-neuronal-cell-derived exosome 110 using a biomarker-specific binding substance on the surface of the exosome.

### «Introduction step»

First, as shown in FIG. 2, a reagent solution L210 is introduced from an introduction port 222 of the fluid device 200 and fed into the flow path 230. The reagent solution L210 is a liquid in which the brain-neuronal-cell-derived exosome 110 and the amyloid-β-specific binding substance 120, labeled with a nucleic acid fragment 121 is dispersed, and also contains a reagent for detecting the nucleic acid fragment 121. In a case where amyloid-β 111 binds to the brain-neuronal-cell-derived exosome 110, the amyloid-β-specific binding substance 120 labeled with the nucleic acid fragment 121 binds to the amyloid-β 111 bound to the brain-neuronal-cell-derived exosome 110.

The reagent solution L210 fed into the flow path 230 comes into contact with the well array 240. Then, the reagent solution L210 is accommodated inside the well 241. As a result, the brain-neuronal-cell-derived exosome 110, the amyloid-β-specific binding substance 120 labeled with the nucleic acid fragment 121, and the reagent for detecting the nucleic acid fragment 121 are introduced into the well 241.

The number of the brain-neuronal-cell-derived exosomes 110 to be introduced into one well 241 is not particularly limited, but preferably, one or fewer, that is, 0 or 1, brain-neuronal-cell-derived exosomes 110 are introduced into one well 241. With this, the detection of amyloid-β 111 bound to the brain-neuronal-cell-derived exosomes 110 can be carried out in one unit, that is, digital measurement is possible. In addition, it is not necessary to introduce the brain-neuronal-cell-derived exosomes 120 into all the wells of the well array.

Means for introducing the brain-neuronal-cell-derived exosomes 110 into the well 241 is not particularly limited, and it is possible to select appropriate means according to the selected brain-neuronal-cell-derived exosomes 110. Examples thereof include a method in which the brain-neuronal-cell-derived exosomes 110 are precipitated in a fluid device (in a flow path) by their own weight and distributed to the wells 241. Alternatively, the biomarker-specific binding substance on the surface of the exosome may be bound to the brain-neuronal-cell-derived exosomes 110 that are not easily precipitated by their own weight for feeding using the above-described biomarker-specific binding substance on the surface of the exosome, which can capture the brain-neuronal-cell-derived exosomes 110, or introduction efficiency of the brain-neuronal-cell-derived exosomes 110 into the well 241 can be improved by fixing the biomarker-specific binding substance on the surface of the exosome to the well 241 in advance, and capturing the fed brain-neuronal-cell-derived exosomes 110.

The step of binding the biomarker-specific binding substance on the surface of the exosome to the biomarker 112 present on the surface of the brain-neuronal-cell-derived exosomes 110 and capturing the brain-neuronal-cell-derived exosomes 110 can be carried out at any time point of the detection method of the present embodiment. For example, this step may be carried out by bringing the brain-neuronal-cell-derived exosomes 110 into contact with the biomarker-specific binding substance on the surface of the exosome in a sample tube before the step of introducing the brain-neuronal-cell-derived exosomes 110 into the well 241. Alternatively, after the biomarker-specific binding substance on the surface of the exosome is introduced into the well 241, the brain-neuronal-cell-derived exosomes 110 may be introduced into the well 241, and the biomarker-specific binding substance on the surface of the exosome and the brain-neuronal-cell-derived exosomes 110 may be brought into contact with each other in the well 241.

The biomarker-specific binding substance on the surface of the exosome may be a conjugate of a solid phase and the biomarker-specific binding substance on the surface of the exosome.

Examples of the solid phase include particles, films, substrates, and the like. In addition, the biomarker-specific binding substance on the surface of the exosome may be one type, or may be two or more types. For example, the biomarker-specific binding substance on the surface of the exosome may be three types, may be four types, or may be five or more types.

The particle is not particularly limited, and examples thereof include a polymer particle, a magnetic particle, a glass particle, and the like. The particle is preferably a particle subjected to a surface treatment to avoid non-specific adsorption. In addition, to fix the specific binding substance, a particle having a functional group such as a carboxyl group on the surface is preferable. More specifically, the product named "Magnosphere LC300" manufactured by JSR Corporation and the like can be used.

Examples of the amyloid-β-specific binding substance 120 and the biomarker-specific binding substance on the surface of the exosome include an antibody, an antibody fragment, an aptamer, and the like. Examples of the antibody fragment include Fab, F(ab')₂, Fab', a single chain antibody (scFv), a disulfide-stabilized antibody (dsFv), a dimeric V-region fragment (Diabody), a peptide including CDR, and the like. An antibody may be a monoclonal antibody, or may be a polyclonal antibody. In addition, a commercially available antibody may also be used.

As a biomarker-specific binding substance on the surface of the exosome, for example, since ganglioside GM1 is present as a biomarker on the surface of the brain-neuronal-cell-derived exosome, the biomarker-specific binding substance on the surface of the exosome may be an antibody, an antibody fragment, an aptamer, a protein, or the like, which specifically binds to ganglioside GM1. Examples of the protein which specifically binds to the ganglioside GM1 include a cholera toxin subunit B (hereinafter, also referred to as CTB), and the like.

Examples of the method of labeling the amyloid-β-specific binding substance with a nucleic acid fragment include a method of using a crosslinking agent. The amyloid-β-specific binding substance may be labeled with the nucleic acid fragment through a molecule that is a linker. The linker is not particularly limited, and examples thereof include a polyethylene chain, a hydrocarbon chain, a peptide, and the like. The nucleic acid fragment may be DNA, or may be RNA. In addition, an artificial nucleic acid such as BNA or LNA may be contained.

In addition, for example, in a case where an antibody molecule is labeled with a nucleic acid fragment, using the antibody molecule as amyloid-β-specific binding substance, since the nucleic acid fragment is a relatively small molecule, for example, several to several tens of nucleic acid fragments per antibody molecule can be labeled. By increasing the number of the nucleic acid fragments per antibody molecule, the time required to detect the amyloid-β-specific binding substance can be further reduced. In addition, in a case where an antibody molecule is labeled with a nucleic acid fragment, several to several hundreds of nucleic acid fragments per antibody molecule may be labeled.

According to the present embodiment, for example, the time for the nucleic acid detection reaction (for example, ICA reaction) is about 1 minute to 30 minutes, and thus rapid nucleic acid detection can be performed.

The method of fixing a biomarker-specific binding substance on a surface of an exosome on a particle surface is not particularly limited, and examples thereof include a method using physical adsorption, a method using chemical binding, a method using avidin-biotin binding, a method of using binding of protein G or protein A and an antibody, and the like. Examples of the method using physical adsorption include a method in which a biomarker-specific binding substance on a surface of an exosome is fixed to a particle surface by hydrophobic interaction or electrostatic interaction. Examples of the method using chemical binding include a method using a crosslinking agent. For example, in a case where the surface of the particle has a hydroxyl group, a crosslinking agent is reacted with a carboxyl group of a biomarker-specific binding substance on a surface of an exosome to be actively esterified, and then the hydroxyl group is reacted with the ester group, which enables the biomarker-specific binding substance on the surface of the exosome to be fixed on the particle surface. In addition, it is preferable to provide a spacer between the biomarker-specific binding substance on the surface of the exosome and the particle surface so that recognition ability of the brain-neuronal-cell-derived exosome by the biomarker-specific binding substance on the surface of the exosome is not inhibited.

As described above, in a case where the brain-neuronal-cell-derived exosome 110 is introduced into the well 241 using the biomarker-specific binding substance on the surface of the exosome, a conjugate between the biomarker-specific binding substance on the surface of the exosome and the brain-neuronal-cell-derived exosome 110 is preferably formed under a condition under which 0 or 1 brain-neuronal-cell-derived exosomes 110 are detected in one biomarker-specific binding substance on a surface of an exosome. In addition, it is preferably configured such that 0 or 1 biomarker-specific binding substances on the exosome surface are introduced into one well 241. This makes digital measurement possible.

When the brain-neuronal-cell-derived exosome 110 is brought into contact with the amyloid-β-specific binding substance 120 labeled with the nucleic acid fragment 121, in a case where the amyloid- β 111 binds to the brain-neuronal-cell-derived exosome 110, the amyloid-β 111 bound to the brain-neuronal-cell-derived exosome 110 and the amyloid-β-specific binding substance 120 labeled with the nucleic acid fragment 121 bind to each other to form a complex 100. The formation of the complex 100 may be carried out in the sample tube, or may be carried out in the well 241.

### «Encapsulation step»

A step of sealing an opening portion of the well 241, after introducing the brain-neuronal-cell-derived exosome 110, the amyloid-β-specific binding substance 120 labeled with the nucleic acid fragment 121, and the like into the well 241 may be carried out.

A method of sealing the opening portion of the well 241 is not particularly limited as long as there can be a state in which a liquid accommodated inside a well 241 is not mixed with a liquid accommodated inside any other well 241, and for example, sealing may be carried out by covering the opening portion of the well 241 with a sealing liquid. Alternatively, the opening may be sealed by laminating a plate-like member such as a glass plate on the opening portion of the well 241.

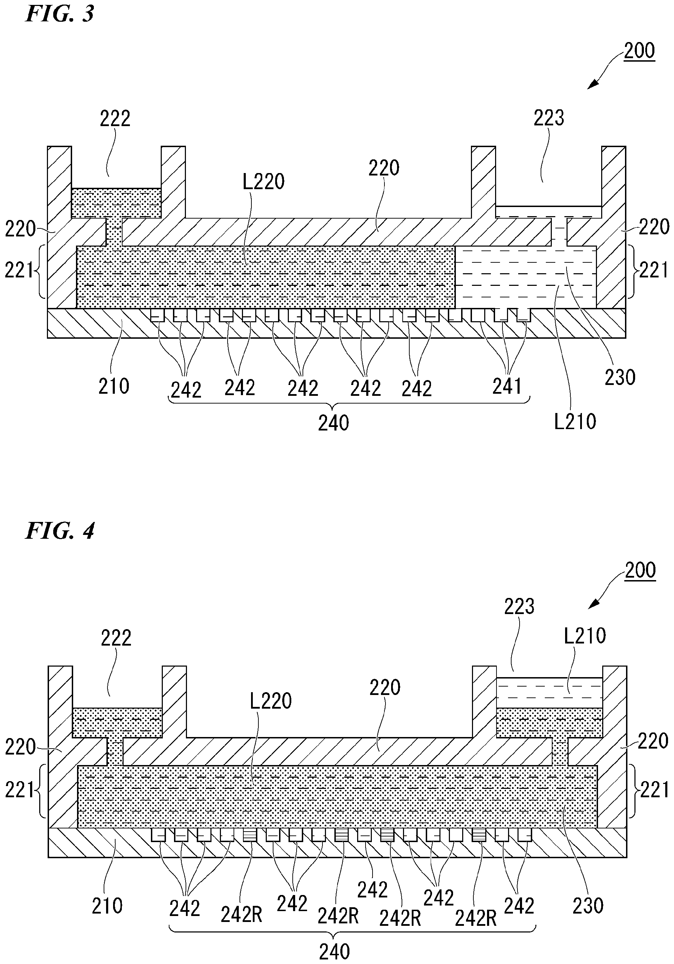

For example, as shown in FIG. 3, a sealing liquid L220 is fed from the introduction port 222 of the lid member 220 to the flow path 230 between the substrate 210 and the lid member 220. The sealing liquid L220 fed into the flow path 230 comes into contact with the well array 240. Then, the sealing liquid L220 pushes out and replaces the reagent solution L210 that is not accommodated in the well 241 among the reagent solutions L210 fed into the flow path 230. With this, the sealing liquid L220 individually seals the plurality of wells 241 accommodating the reagent solution L210 containing the brain-neuronal-cell-derived exosomes 110, and the well 241 becomes an independent reaction space (micro-compartment 242). In a case where the flow path 230 is filled with the sealing liquid L220, excess sealing liquid L220 is discharged from the discharge port 223. FIG. 4 shows a state in which all the wells 241 of the well array 240 are sealed with the sealing liquid L220, and a sealed well (micro-compartment) 242 is formed.

In addition, by dissolving a lipid in the reagent solution L210, feeding the sealing liquid L220 to the flow path 230, and then feeding the liquid containing the lipid again, a lipid bilayer membrane can be formed in the opening portion of the well 241, the plurality of wells 241 can be individually sealed by the lipid bilayer membrane, and the sealed well 242 can also be formed. Examples of the lipid forming the lipid bilayer membrane include 1,2-dioleoyl-sn-glycero-3-phosphoethanolamine (DOPE), 1,2-dioleoyl-sn-glycero-3-phosphoglycerol (DOPG), a mixture thereof, and the like, but are not limited thereto.

The wells 242 sealed by any of the sealing methods described above may contain the amyloid-β-specific binding substance 120 not bound to the brain-neuronal-cell-derived exosome 110.

The sealing liquid is a liquid capable of forming liquid droplets (micro-droplets) by individually sealing each liquid introduced into the plurality of wells 241 so that they do not mix with each other, and is preferably an oleaginous solution, and more preferably an oil. As the oil, a fluorine-based oil, a silicone-based oil, a hydrocarbon-based oil, a mixture thereof, and the like can be used. More specifically, the product named "FC-40" manufactured by Sigma-Aldrich Co., LLC can be used. FC-40 (CAS number: 86508-42-1) is a fluorinated aliphatic compound and has a specific gravity of 1.85 g/mL at 25°C.

### <<Detection Step>>

Subsequently, the nucleic acid fragment 121 is detected. Detection of the nucleic acid fragment 121 is preferably carried out using a signal amplification reaction. Examples of the signal amplification reaction include an invasive cleavage assay (ICA).

The ICA reaction is related to a principle that signal amplification proceeds through a cycle of two reactions of (1) complementary binding between nucleic acids and (2) recognition and cleavage of a triple-stranded structure by an enzyme.

The ICA reaction is less affected by reaction cycle inhibition due to impurities. Therefore, by using the ICA reaction, the brain-neuronal-cell-derived exosome 110 can be detected with high accuracy. In a case where the ICA reaction is used for the signal amplification reaction, the reagent solution L210 (the liquid containing the brain-neuronal-cell-derived exosome 110 and the amyloid-β-specific binding substance 120) contains a reaction reagent necessary for the ICA reaction.

Examples of the reaction reagent necessary for the ICA reaction include ICA reaction reagents such as a flap probe, an invasion probe, a flap endonuclease (FEN), and a fluorescent substrate. The flap probe and the invasion probe are nucleic acid fragments designed to hybridize to the nucleic acid fragment 121 to form a flap structure with the nucleic acid fragment 121.

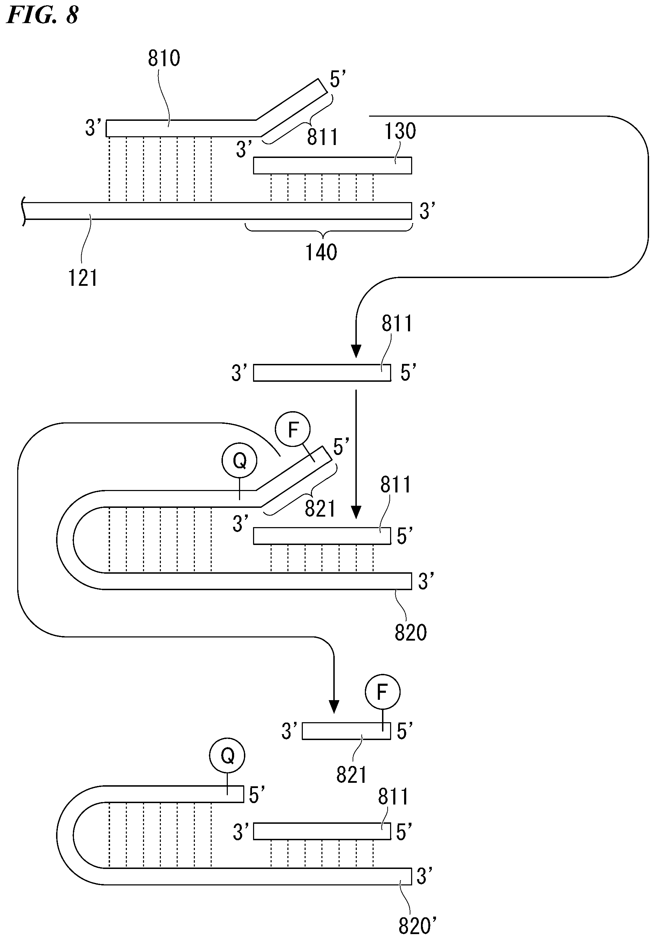

FIG. 8 is a schematic diagram showing an example of the ICA method. First, the flap probe and the invasion probe are hybridized to the nucleic acid fragment 121. In an example of FIG. 8, a flap probe 810 and an invasion probe 130 are hybridized to the nucleic acid fragment 121. As a result, a first flap site 811 is formed.

Subsequently, when an FEN is reacted with the first flap site 811, the first flap site 811 is cleaved, and the nucleic acid fragment 811 is generated. Subsequently, the nucleic acid fragment 811 is hybridized to a fluorescent substrate (a nucleic acid fragment 820) to form a second flap site 821.

In the example of FIG. 8, a fluorescent substance F is bound to a 5' terminal of the nucleic acid fragment 820, and a quenching substance Q is bound to several bases 3' side from the 5' terminal of the nucleic acid fragment 820. Subsequently, when the FEN is reacted with the second flap site 821, the second flap site 821 is cleaved, and the nucleic acid fragment 821 is generated. As a result, the fluorescent substance F is separated from the quenching substance Q, and a fluorescent signal is generated. The nucleic acid fragment 121 can be detected by detecting this fluorescent signal.

As the reagent solution L210, a general liquid used in biochemical analysis carried out using a fluid device can be used, and an aqueous solution is preferable. In addition, by the reagent solution L210 containing a surfactant and the like, a liquid may be easily sealed in the well.

In a case where the ICA reaction is used for detecting the nucleic acid fragment 121, when the nucleic acid fragment 121 is present, the fluorescent substance F is released from the quenching substance Q by the enzymatic reaction by an isothermal reaction, and a predetermined fluorescent signal is emitted in response to excitation light.

For detection of the nucleic acid fragment 121, a known appropriate method can be selected depending on the type of the signal to be detected. For example, in a case where a fluorescent signal is observed, the well 242 is irradiated with excitation light corresponding to the fluorescent substance, and the fluorescence emitted by the fluorescent substance is observed. For example, as shown in FIG. 4, a predetermined reaction is carried out in the sealed well 242, and the generated signal is observed. A well 242R is a well in which a signal is detected, and the well 242 is a well in which no signal is detected.

Next, with reference to FIGS. 5 to 7, the method of the present embodiment will be described by taking a case where the fluid device 500 is used as an example.

First, as shown in FIG. 5, the reagent solution L210 is introduced into the fluid device 500. The reagent solution L210 is a liquid in which the brain-neuronal-cell-derived exosome 110 and the amyloid-β-specific binding substance 120 labeled with the nucleic acid fragment 121 are dispersed, and also contains a reagent for detecting the nucleic acid fragment 121. In the reagent solution L210, a concentration of the brain-neuronal-cell-derived exosome 110 is preferably adjusted to a concentration at which one or fewer molecules of the brain-neuronal-cell-derived exosome 110 per well are introduced into the well 241.

Subsequently, as shown in FIG. 6, a sealing liquid L220 is introduced into the fluid device 500. A specific gravity of the sealing liquid L220 is larger than that of the reagent solution L210. Therefore, the sealing liquid L220 sinks below the reagent solution L210 that is not accommodated in the well 241, out of the reagent solution L210, and comes into contact with the well array 240. Then, the sealing liquid L220 individually seals each of the plurality of wells 241 accommodating the reagent solution L210 containing the brain-neuronal-cell-derived exosome to become an independent reaction space (micro-compartment 242).

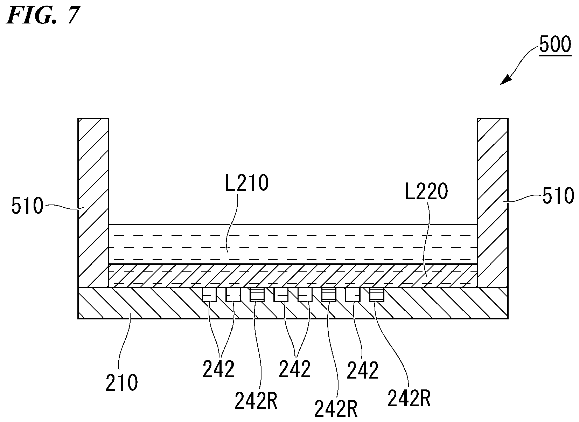

Subsequently, as shown in FIG. 7, a predetermined reaction is carried out in the wells 242, and the generated signal is observed. A well 242R is a well in which a signal is detected, and the well 242 is a well in which no signal is detected.

### <Second embodiment>

In one embodiment, the present invention provides a method for detecting amyloid-β bound to a brain-neuronal-cell-derived exosome in blood, the method including a step of binding a biomarker present on a surface of the exosome to a substance (a biomarker-specific binding substance on a surface of the exosome) that specifically binds to the biomarker present on the surface of the exosome, labeled with a nucleic acid fragment; a step of detecting amyloid-β bound to the brain-neuronal-cell-derived exosome in blood; and a step of detecting the biomarker-specific binding substance on the surface of the exosome. Hereinafter, a detection method according to the present embodiment will be described.

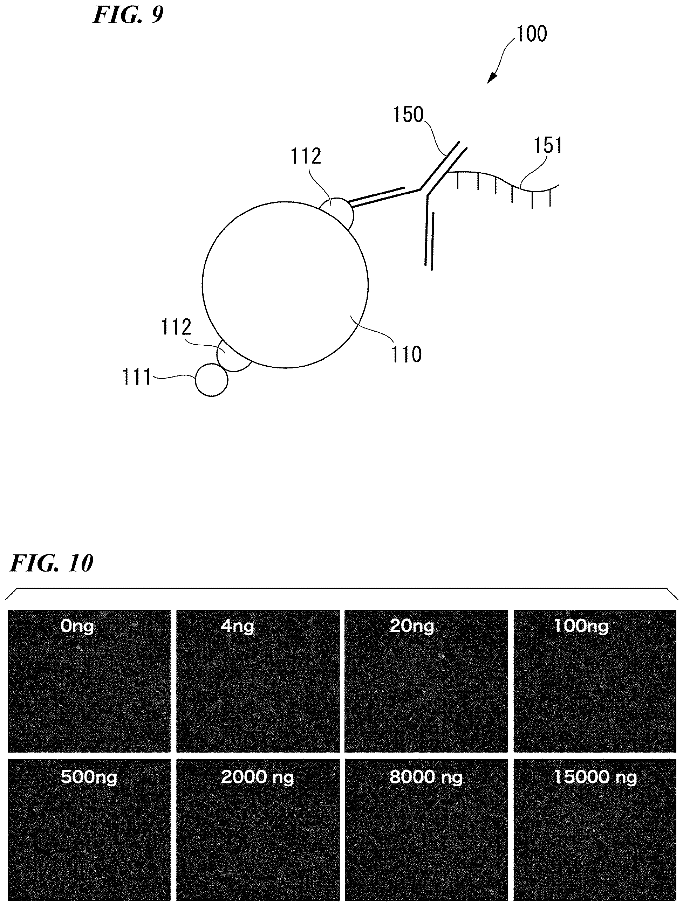

FIG. 9 is a schematic diagram showing a detection method of the present embodiment. As shown in FIG. 9, in the detection method of the present embodiment, first, the biomarker 112 present on the surface of the brain-neuronal-cell-derived exosome, and the biomarker-specific binding substance on the surface of the exosome 150 that specifically binds to the biomarker 112 present on the surface of the brain-neuronal-cell-derived exosome, labeled with the nucleic acid fragment 151, are bound to each other. The amyloid-β 111 binds to a biomarker 112 present on a surface of the brain-neuronal-cell-derived exosome 110. The biomarker-specific binding substance 150 on the surface of the exosome binds to the exosome-specific biomarker 112 to which amyloid-β 111 is not bound.

Examples of the biomarker 112 present on a membrane surface of the brain-neuronal-cell-derived exosome 110, to which amyloid-β 111 can be bound, include those described above.

Subsequently, the biomarker-specific binding substance on the surface of the exosome 150, which is bound to the biomarker 112 present on the surface of the neuronal-cell-derived exosome, is detected. In a case where the detection method of the present embodiment is suitable for detection by digital measurement, it is preferable to use a fluid device having a well array in which a plurality of wells is arranged. As the fluid device, the fluid device described in the first embodiment is used. However, instead of an amyloid-β-specific binding substance labeled with a nucleic acid fragment, a biomarker-specific binding substance on the surface of the exosome labeled with a nucleic acid fragment is used.

Next, the amyloid-β 111 bound to the biomarker 112 present on the surface of the brain-neuronal-cell-derived exosome is detected. The step of detecting amyloid-β 111 bound to the biomarker 112 present on the surface of the brain-neuronal-cell-derived exosome can be carried out by detecting amyloid-β 111 bound to the brain-neuronal-cell-derived exosome 110 using the amyloid-β-specific binding substance 120 that specifically binds to amyloid-β. In a case where amyloid-β 111 binds to the biomarker 112 present on the surface of the brain-neuronal-cell-derived exosome, the brain-neuronal-cell-derived exosome 110 can be captured using the amyloid-β-specific binding substance 120, and thus detection of amyloid-β bound to the brain-neuronal-cell-derived exosome 110 can be carried out by capturing the brain-neuronal-cell-derived exosome 110 using the amyloid-β-specific binding substance 120.

### «Introduction step»

First, as shown in FIG. 2, a reagent solution L210 is introduced from an introduction port 222 of the fluid device 200 and fed into the flow path 230. The reagent solution L210 is a liquid in which the brain-neuronal-cell-derived exosome 110 and the biomarker-specific binding substance on the surface of the exosome 150 labeled with the nucleic acid fragment 151 is dispersed, and also contains a reagent for detecting the nucleic acid fragment 151. In a case where amyloid-β 111 binds to the brain-neuronal-cell-derived exosome 110, the biomarker-specific binding substance on the surface of the exosome 150 binds to the biomarker 112 on the surface of the brain-neuronal-cell-derived exosome 110, to which amyloid-β 111 is not bound.

The reagent solution L210 fed into the flow path 230 comes into contact with the well array 240. Then, the reagent solution L210 is accommodated inside the well 241. As a result, the brain-neuronal-cell-derived exosome 110, the biomarker-specific binding substance on the surface of the exosome 150 labeled with the nucleic acid fragment 151, and the reagent for detecting the nucleic acid fragment 151 are introduced into the well 241.

The number of the brain-neuronal-cell-derived exosomes 110 to be introduced into one well 241 is not particularly limited, but preferably, one or fewer, that is, 0 or 1, brain-neuronal-cell-derived exosomes 110 are introduced into one well 241. With this, the detection of amyloid-β 111 bound to the brain-neuronal-cell-derived exosomes 110 can be carried out in one unit, that is, digital measurement is possible. In addition, it is not necessary to introduce the brain-neuronal-cell-derived exosomes 120 into all the wells of the well array.

Means for introducing the brain-neuronal-cell-derived exosomes 110 into the well 241 is not particularly limited, and it is possible to select appropriate means according to the selected brain-neuronal-cell-derived exosomes 110. Examples thereof include a method in which the brain-neuronal-cell-derived exosomes 110 are precipitated in a fluid device (in flow path) by their own weight and distributed to the wells 241. Alternatively, the amyloid-β-specific binding substance 120 may be bound to the brain-neuronal-cell-derived exosomes 110 that are not easily precipitated by their own weight for feeding using the above-described amyloid-β-specific binding substance 120, which can capture the brain-neuronal-cell-derived exosomes 110, or introduction efficiency of the brain-neuronal-cell-derived exosomes 110 into the well 241 can be improved by fixing the amyloid-β-specific binding substance 120 to the well 241 in advance, and capturing the fed brain-neuronal-cell-derived exosomes 110.

A step of causing the amyloid-β-specific binding substance 120 to bind to amyloid-β 111 bound to the brain-neuronal-cell-derived exosome 110 to capture the brain-neuronal-cell-derived exosome 110 can be carried out at any time point in the detection method of the present embodiment. For example, this step may be carried out by bringing the brain-neuronal-cell-derived exosome 110 and the amyloid-β-specific binding substance 120 into contact with each other in the sample tube before the step of introducing the brain-neuronal-cell-derived exosome 110 into the well 241. Alternatively, after the amyloid-β-specific binding substance 120 is introduced into the well 241, the brain-neuronal-cell-derived exosome 110 is introduced into the well 241, and the amyloid-β-specific binding substance 120 and the brain-neuronal-cell-derived exosome 110 are brought into contact with each other in the well 241.

The amyloid-β-specific binding substance may be a conjugate of a solid phase and the amyloid-β-specific binding substance.

Regarding the solid phase, the amyloid-β-specific binding substance, and the biomarker-specific binding substance on the surface of the exosome, the same ones as those described in the first embodiment are used.

Examples of the method of labeling a biomarker-specific binding substance on a surface of an exosome with a nucleic acid fragment include a method of using a crosslinking agent. The amyloid-β-specific binding substance may be labeled with the nucleic acid fragment through a molecule that is a linker. The linker is not particularly limited, and examples thereof include a polyethylene chain, a hydrocarbon chain, a peptide, and the like. The nucleic acid fragment may be DNA, or may be RNA. In addition, an artificial nucleic acid such as BNA or LNA may be contained.

In addition, for example, in a case where an antibody molecule is labeled with a nucleic acid fragment using the antibody molecule as the biomarker-specific binding substance on the surface of the exosome, since the nucleic acid fragment is relatively small, several to several tens of nucleic acid fragments, for example, per antibody molecule can be labeled. By increasing the number of nucleic acid fragments per antibody molecule, the time required to detect the biomarker-specific binding substance on the surface of the exosome can be further reduced. In addition, in a case where an antibody molecule is labeled with a nucleic acid fragment, several to several hundreds of nucleic acid fragments per antibody molecule may be labeled.

According to the present embodiment, for example, the time for the nucleic acid detection reaction (for example, ICA reaction) is about 1 minute to 30 minutes, and thus rapid nucleic acid detection can be performed.

The method of fixing an amyloid-β-specific binding substance on a particle surface is not particularly limited, and examples thereof include a method using physical adsorption, a method using chemical binding, a method using avidin-biotin binding, a method using binding of protein G or protein A and an antibody, and the like. Examples of the method using physical adsorption include a method in which the amyloid-β-specific binding substance is fixed on the particle surface by hydrophobic interaction or electrostatic interaction. Examples of the method using chemical binding include a method using a crosslinking agent. For example, in a case where the surface of the particle has a hydroxyl group, a crosslinking agent is reacted with a carboxyl group of an amyloid-β-specific binding substance to be actively esterified, and then the hydroxyl group is reacted with the ester group, which enables the amyloid-β-specific binding substance to be fixed on the particle surface. In addition, it is preferable to provide a spacer between the amyloid-β-specific binding substance and the particle surface so that the recognition ability of the brain-neuronal-cell-derived exosome by the amyloid-β-specific binding substance is not inhibited.

As described above, in a case where the brain-neuronal-cell-derived exosome 110 is introduced into the well 241 using the amyloid-β-specific binding substance 120, a conjugate between the amyloid-β-specific binding substance and the brain-neuronal-cell-derived exosome 110 is preferably formed under a condition under which 0 or 1 brain-neuronal-cell-derived exosomes 110 are detected in one amyloid-β-specific binding substance. In addition, it is preferable that the well 241 be configured such that 0 or 1 units of the amyloid-β-specific binding substance are introduced into one well 241. This makes digital measurement possible.

In a case where amyloid-β 111 binds to the brain-neuronal-cell-derived exosome 110, the amyloid-β 111 bound to the brain-neuronal-cell-derived exosome 110 and the amyloid-β-specific binding substance 120 are bound and introduced into the well 241.

Subsequently, in a case where the brain-neuronal-cell-derived exosome 110 and the biomarker-specific binding substance on the surface of the exosome 150 labeled with the nucleic acid fragment 151 are brought into contact with each other, the brain-neuronal-cell-derived exosome 110 and the biomarker-specific binding substance on the surface of the exosome 150 labeled with the nucleic acid fragment 151 are bound to form the complex 100. The formation of the complex 100 may be carried out in the sample tube, or may be carried out in the well 241.

### «Encapsulation step»

After introducing the brain-neuronal-cell-derived exosome 110, the biomarker-specific binding substance on the surface of the exosome 150 labeled with the nucleic acid fragment 151, and the like into the well 241, the encapsulation step can be carried out in the same manner as in the first embodiment described above.

### <<Detection step>>

Subsequently, the nucleic acid fragment 151 is detected. The detection of the nucleic acid fragment 151 can be carried out in the same manner as in the first embodiment, except that the nucleic acid fragment 151 is used instead of the nucleic acid fragment 121.

Next, with reference to FIGS. 5 to 7, the method of the present embodiment will be described by taking a case where the fluid device 500 is used as an example.

First, as shown in FIG. 5, the reagent solution L210 is introduced into the fluid device 500. The reagent solution L210 is a liquid in which the brain-neuronal-cell-derived exosome 110 and the biomarker-specific binding substance on the surface of the exosome 150 labeled with the nucleic acid fragment 151, are dispersed, and also contains a reagent for detecting the nucleic acid fragment 151. In the reagent solution L210, a concentration of the brain-neuronal-cell-derived exosome 110 is preferably adjusted to a concentration at which one or fewer molecules of the brain-neuronal-cell-derived exosome 110 per well are introduced into the well 241.

Subsequently, as shown in FIG. 6, a sealing liquid L220 is introduced into the fluid device 500. A specific gravity of the sealing liquid L220 is larger than that of the reagent solution L210. Therefore, the sealing liquid L220 sinks below the reagent solution L210 that is not accommodated in the well 241, out of the reagent solution L210, and comes into contact with the well array 240. Then, the sealing liquid L220 individually seals each of the plurality of wells 241 accommodating the reagent solution L210 containing the brain-neuronal-cell-derived exosome to become an independent reaction space (micro-compartment 242).

Subsequently, as shown in FIG. 7, a predetermined reaction is carried out in the wells 242, and the generated signal is observed. A well 242R is a well in which a signal is detected, and the well 242 is a well in which no signal is detected.

### [Kit]

In one embodiment, the present invention provides a kit for detection of amyloid-β 111 bound to a brain-neuronal-cell-derived exosome 110 in blood, the kit including an amyloid-β-specific binding substance 120 labeled with a nucleic acid fragment 121.

With the kit of the present embodiment, it is possible to suitably carry out the above-described method for detecting a brain-neuronal-cell-derived exosome of the first embodiment. Therefore, it can be said that the kit of the present embodiment is for use in the method for detecting amyloid-β 111 bound to a brain-neuronal-cell-derived exosome 110 in blood, the method including a step of binding amyloid-β 111 bound to the brain-neuronal-cell-derived exosome 110 in blood to an amyloid-β-specific binding substance 120 that specifically binds to the amyloid-β, labeled with a nucleic acid fragment 121; and a step of detecting the amyloid-β-specific binding substance 120.

In the kit of the present embodiment, the brain-neuronal-cell-derived exosome, the amyloid-β-specific binding substance, and the nucleic acid fragment are the same as those described above.

The kit of the present embodiment may include a well array 240 having a plurality of wells 241. In addition, the kit of the present embodiment may further include a sealing liquid L220 for sealing an opening portion of the well 241. The sealing liquid L220 is the same as described above.

In addition, the kit of the present embodiment may further include a substance that detects a biomarker 112 present on a surface of the brain-neuronal-cell-derived exosome 110. Examples of the substance that detects the biomarker 112 present on the surface of the brain-neuronal-cell-derived exosome 110 include the above-described biomarker-specific binding substance on the surface of the exosome.

In addition, the kit of the present embodiment may further include a reagent for detecting the nucleic acid fragment 121.

Examples of such a reagent include the above-described reagent for an ICA reaction, and specific examples thereof include a flap probe, an invasion probe, a flap endonuclease (FEN), a fluorescent substrate, and the like.

In one embodiment, the present invention provides a kit for detection of amyloid-β bound to a brain-neuronal-cell-derived exosome in blood, the kit including: a biomarker-specific binding substance on a surface of an exosome that specifically binds to a biomarker present on a surface of the brain-neuronal-cell-derived exosome, labeled with a nucleic acid fragment, and an amyloid-β-specific binding substance that specifically binds to amyloid-β.

With the kit of the present embodiment, it is possible to suitably carry out the above-described method for detecting a brain-neuronal-cell-derived exosome of the second embodiment. Therefore, it can be said that the kit of the present embodiment is for use in a method for detecting amyloid-β 111 bound to a brain-neuronal-cell-derived exosome 110 in blood, the method including: a step of binding a biomarker 112 present on a surface of the exosome 110 to a biomarker-specific binding substance on a surface of an exosome 150 that specifically binds to the biomarker present on the surface of the exosome, labeled with a nucleic acid fragment 151; a step of detecting amyloid-β 111 bound to the brain-neuronal-cell-derived exosome 110 in blood; and a step of detecting the biomarker-specific binding substance on the surface of the exosome 150.

In the kit of the present embodiment, the brain-neuronal-cell-derived exosome, the amyloid-β-specific binding substance, the biomarker-specific binding substance on the surface of the exosome, and the nucleic acid fragment are the same as those described above.

The kit of the present embodiment may include a well array 240 having a plurality of wells 241. In addition, the kit of the present embodiment may further include a sealing liquid L220 for sealing an opening portion of the well 241. The sealing liquid L220 is the same as described above.

In addition, the kit of the present embodiment may further include a reagent for detecting the nucleic acid fragment 151.

Examples of such a reagent include the above-described reagent for an ICA reaction, and specific examples thereof include a flap probe, an invasion probe, a flap endonuclease (FEN), a fluorescent substrate, and the like.

### [Method for evaluating accumulation level of amyloid-β in brain]

In one embodiment, the present invention provides a method for evaluating an accumulation level of amyloid-β in a brain, the method including: a step of detecting amyloid-β bound to a brain-neuronal-cell-derived exosome in blood; and a step of evaluating an accumulation level of amyloid-β in the brain based on the detected amount of the amyloid-β.

In the evaluation method of the present embodiment, in a case where the detected amount of amyloid-β bound to a brain-neuronal-cell-derived exosome is high, the accumulation level of amyloid-β in the brain can be evaluated as high. In addition, in a case where the detected amount of amyloid-β bound to a brain-neuronal-cell-derived exosome is low, the accumulation level of amyloid-β in the brain can be evaluated as low.

Since the accumulation level of amyloid-β in the brain can be evaluated by the evaluation method of the present embodiment, it is possible to carry out early treatment and prevention of Alzheimer's disease of which accumulation of amyloid-β in the brain is considered to be a cause. For example, in a case where it is determined that the accumulation level of amyloid-β in the brain is high by the evaluation method of the present embodiment, treatment of Alzheimer's disease can be started.

In the evaluation method of the present embodiment, for the detection of amyloid-β bound to the brain-neuronal-cell-derived exosome in blood, the above-described method for detecting amyloid-β bound to the brain-neuronal-cell-derived exosome in blood can be used. Therefore, with the evaluation method of the present embodiment, it is possible to rapidly and accurately evaluate the accumulation level of amyloid-β in the brain.

In addition, the evaluation method of the present embodiment enables the accumulation level of amyloid-β in the brain to be evaluated by simply collecting blood from a subject, is not invasive, unlike the cerebrospinal fluid examination, and does not require any special equipment such as a PET imaging method.

### EXAMPLES

The present invention will be described below with reference to examples, but is not limited to the following examples.

### [Experimental Example 1]

### <Reagent preparation>

### (Brain-neuronal-cell-derived exosome-containing sample)

In a sample containing a brain-neuronal-cell-derived exosome to which amyloid-β is bound, a culture supernatant obtained by culturing a cell prepared by stably introducing a plasmid embedded with a gene of isoform 751 (APP751) of human amyloid-β precursor protein (hereinafter, also referred to as APP) into a mouse neuro2A (N2a) cell, in a Dulbecco's Modified Eagle culture medium for 24 hours, was used. Exosomes were recovered from the culture supernatant by an ultracentrifugation method. Specifically, the culture supernatant was subjected to centrifugation stepwise at 2000 × g for 10 minutes, at 10,000 × g for 30 minutes, and at 100,000 × g for 70 minutes to recover an exosome, and a sample containing a brain-neuronal-cell-derived exosome was obtained. Recovery of the exosome from the serum was carried out using MagCapture (registered trademark) Exosome Isolation Kit PS (manufactured by Fujifilm Wako Pure Chemical Corporation).

### (Preparation of CTB-fixed magnetic beads)

To fix the cholera toxin subunit B (CTB, manufactured by Sigma) on the magnetic beads, CTB was added to a solution of carboxyl group-modified magnetic beads (Magnosphere, LC300, manufactured by JSR Corporation), mixed to 100 µL, reacted with a rotator for 30 minutes, and furthermore, reacted with a condensing agent EDC for 3 hours to carry out fixation of CTB on the carboxyl magnetic beads.

To remove unreacted CTB and reagents after the reaction, magnetic collection of the CTB-fixed carboxyl magnetic beads using a magnetic stand and washing with PBS-T (PBS containing 0.1% Tween 20) were repeated 3 times, and CTB-fixed carboxyl magnetic beads (CTB beads) were prepared.

### (Nucleic acid for modifying anti-amyloid-β antibody and nucleic acid for detection)

As the nucleic acid for modifying an anti-amyloid-β antibody and for nucleic acid detection reaction, the following nucleic acids were used.
- DNA fragment 1: has a base sequence set forth in SEQ ID NO: 1.
- Flap probe 1: has a base sequence set forth in SEQ ID NO: 2.
- Invasion probe 1: has a base sequence set forth in SEQ ID NO: 3.

### (Preparation of nucleic acid-modified anti-amyloid-β antibody)

A DNA fragment (DNA fragment 1) having a base sequence set forth in SEQ ID NO: 1 was bound to an anti-amyloid-β monoclonal antibody (model "BAN50", manufactured by Fujifilm Wako Pure Chemical Corporation) to produce a nucleic acid-labeled anti-amyloid-β antibody. A commercially available kit (the product named "Protein-Oligo Conjugation Kit", manufactured by Solulink Inc.) was used for binding of the DNA fragment. The nucleic acid-labeled anti-amyloid-β antibody was prepared so that 5 to 20 times the amount of nucleic acid was modified with respect to the antibody (one antibody molecule).

### (Preparation of nucleic acid detection reagent)

To carry out detection of a nucleic acid-modified antibody by the ICA reaction, an ICA reaction reagent was prepared as a nucleic acid detection reagent. The reagent composition of the ICA reaction reagent in the present experimental example is as follows.

The ICA reaction reagent in the present experimental example included 0.1 µM flap probe 1 (SEQ ID NO: 2), 1 µM invasion probe 1 (SEQ ID NO: 3), 2 µM FRET cassette (FAM) (manufactured by Hologic, Inc.), 2 µM FRET cassette (Redmond RED) (manufactured by Hologic, Inc.), 10 mM MOPS (pH 7.9), 10 mM MgCl₂, and 1,000 U/µL cleavase.

In addition, a concentration of each component in these ICA reaction reagents is a final concentration in the ICA reaction reagent used in Experimental Example 1.

### <Reaction of exosomes, CTB-fixed magnetic beads, and nucleic acid-modified anti-amyloid-β antibody>

CTB specifically binds to ganglioside GM1 present on a membrane surface of a brain-neuronal-cell-derived exosome. Therefore, in a case where CTB-fixed magnetic beads are used, it is possible to capture the brain-neuronal-cell-derived exosome. Specifically, as will be described below, a brain-neuronal-cell-derived exosome was captured using CTB-fixed magnetic beads, and amyloid-β bound to the captured brain-neuronal-cell-derived exosome and an amyloid-β-specific binding substance were bound to form a complex.

A sample tube was mixed with 6 × 10⁵ CTB-fixed carboxyl magnetic beads (CTB beads), PBS adjusted to contain 0, 4, 20, 100, 500, 2,000, 8,000, or 15,000 ng of exosomes and nucleic acid-modified anti-amyloid-β antibodies adjusted to 0.1 ng/mL were mixed so that a total amount thereof reached 100 µL, and the mixture was reacted on a rotator for 1 hour at room temperature to form a complex.

After the reaction, the obtained magnetic beads were magnetically collected using a magnetic stand, and washed by repeating an operation of removing the supernatant and adding PBS containing 0.1% Tween (PBS-T) 5 times, and the supernatant was finally removed to obtain a complex.

The obtained complex was washed and diluted to prepare each reaction mixture solution.

### <Preparation of device>

A device was produced by producing a base material provided with a large number of minute wells using COP (cycloolefin polymer) and bonding a COP lid material. A total volume of the wells per 1 cm² was 0.93 µL. A total number of wells used for the measurement was 1,000,000.

### <Feeding of reaction mixture solution> 18 µL of each reaction mixture solution was fed into the device and introduced into each well. Subsequently, 200 µL of FC-40 (manufactured by Sigma) was fed as a sealing liquid to seal each well.

### <Nucleic acid detection reaction>

The device into which each reaction mixture solution had been fed was set on a hot plate and allowed to react at 66°C for 25 minutes. With this, recognition of a nucleic acid fragment modifying an anti-amyloid-β antibody by the flap probe and the invasion probe, cleavage of the flap probe by the FEN, binding of the released flap probe fragment to the FRET Cassette, and cleavage of the FRET Cassette by the FEN proceeded, and a fluorescent signal was emitted from Alexa488.

### <Fluorescence observation in well>

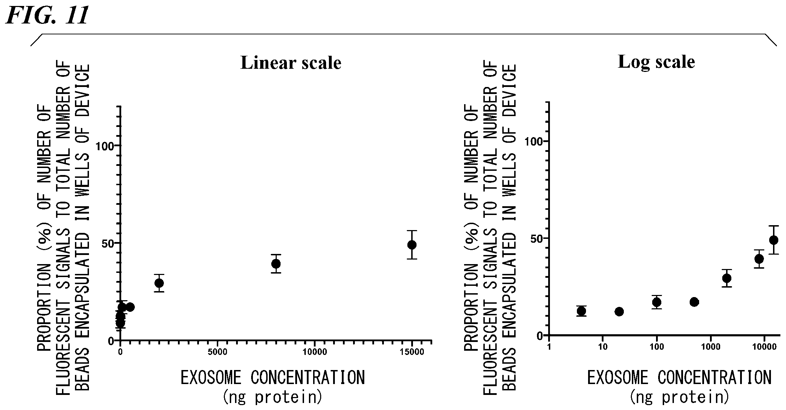

After heating at 66°C for 25 minutes, a fluorescence image of the fluorescent signal obtained from the nucleic acid detection reaction in each well of the device was captured, using a 4X objective lens with a fluorescence microscope (BZ-700, manufactured by KEYENCE CORPORATION) and a GFP fluorescence filter. The exposure time was set to 3,000 msec. FIG. 10 shows the obtained fluorescence image of each reaction mixture solution 1, and FIG. 11 shows a relationship between an exosome concentration and a proportion (%) of the number of fluorescent signals to the total number of beads encapsulated in the wells of the device.

As shown in FIGS. 10 and 11, the fluorescence intensity increased depending on the concentration of exosomes. From these results, it was clarified that amyloid-β bound to the brain-nerve-derived exosome can be detected by the nucleic acid-labeled anti-amyloid-β antibody.

### [Experimental Example 2]

### <Reagent preparation>

### (Brain-neuronal-cell-derived exosome-containing sample)

As a sample containing a brain-neuronal-cell-derived exosome, serum of a 3-month-old or 13-month-old mouse [human APP ^{Sw, Ind} gene-introduced mouse (J20, manufactured by Jackson Laboratory)] into which a human familial mutant APP gene was introduced was used. Recovery of the exosome from the serum was carried out using MagCapture (registered trademark) Exosome Isolation Kit PS (manufactured by Fujifilm Wako Pure Chemical Corporation). PBS was used as a control containing no exosome.

### (Preparation of CTB-fixed magnetic bead, nucleic acid for modifying anti-amyloid-β antibody, nucleic acid for detection, nucleic acid-modified anti-amyloid-β antibody, and nucleic acid detection reagent)

In the same manner as in Experimental Example 1, CTB-fixed magnetic beads, a nucleic acid for modifying an anti-amyloid-β antibody, a nucleic acid for detection, a nucleic acid-modified anti-amyloid antibody, and a nucleic acid detection reagent were prepared.

### <Reaction of exosomes, CTB-fixed magnetic beads, and nucleic acid-modified anti-amyloid-β antibody>

A sample tube was mixed with 6 × 10⁵ CTB-fixed carboxyl magnetic beads (CTB beads), PBS or exosome recovered from each mouse serum of 50, 100, and 250 µL, and nucleic acid-modified anti-amyloid-β antibodies adjusted to 0.1 ng/mL were mixed so that a total amount thereof reached 100 µL, and the mixture was reacted on a rotator for 1 hour at room temperature to form a complex.

After the reaction, the obtained magnetic beads were magnetically collected using a magnetic stand, and washed by repeating an operation of removing the supernatant and adding PBS containing 0.1% Tween (PBS-T) 5 times, and the supernatant was finally removed to obtain a complex.

The obtained complex was diluted with PBS after washing to prepare a reaction mixture solution.

### <Preparation of device and feeding of reaction mixture solution>

Feeding of the reaction mixture solution was carried out in the same procedure as in Experimental Example 1, using the device described in Experimental Example 1.

### <Nucleic acid detection reaction>

The device into which each reaction mixture solution had been fed was set on a hot plate and allowed to react at 66°C for 25 minutes. With this, recognition of a nucleic acid fragment modifying an anti-amyloid-β antibody by the flap probe and the invasion probe, cleavage of the flap probe by the FEN, binding of the released flap probe fragment to the FRET Cassette, and cleavage of the FRET Cassette by the FEN proceeded, and a fluorescent signal was emitted from Alexa488.

### <Measurement of fluorescence intensity>

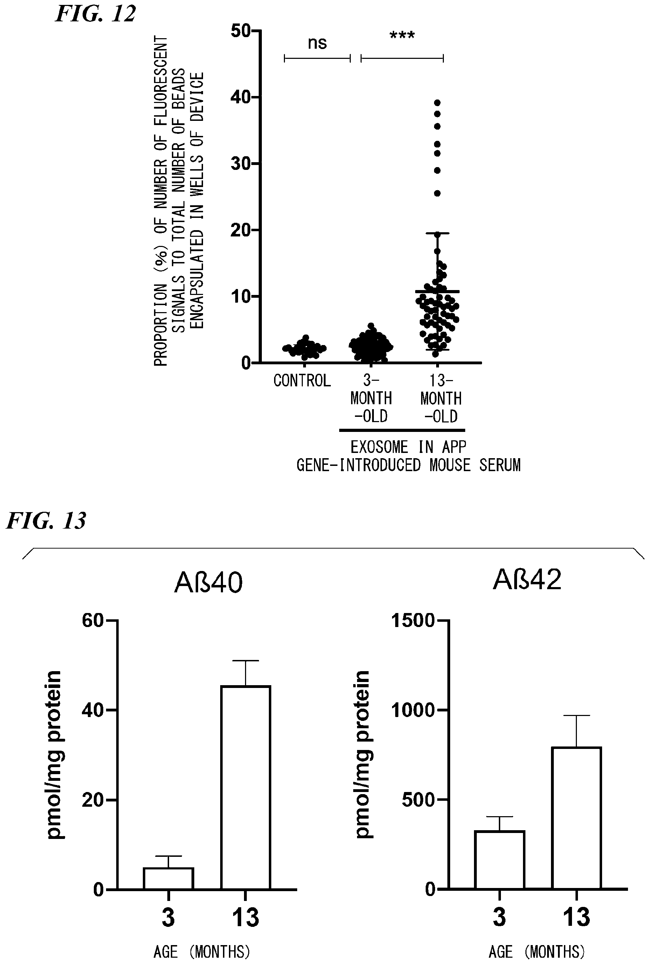

After heating at 66°C for 25 minutes, a fluorescence image of the fluorescent signal obtained from the nucleic acid detection reaction in each well of the device was captured, using a 4X objective lens with a fluorescence microscope (BZ-700, manufactured by KEYENCE CORPORATION) and a GFP fluorescence filter. The exposure time was set to 3,000 msec. FIG. 12 shows a proportion (%) of the number of fluorescent signals in the total number of beads encapsulated in the wells of the device, measured for each reaction mixture solution in the control and the 3-month-old or 13-month-old human familial mutant APP gene-introduced mouse serum.

As shown in FIG. 12, almost no fluorescence was detected from the 3-month-old human familial mutant APP gene-introduced mouse serum as in the control. On the other hand, a significant increase in fluorescence intensity from the serum of the 13-month-old human familial mutant APP gene-introduced mouse was recognized as compared to the serum of the 3-month-old human familial mutant APP gene-introduced mouse.

### [Experimental Example 3]

### (Detection of amyloid-β in mouse brain tissue)

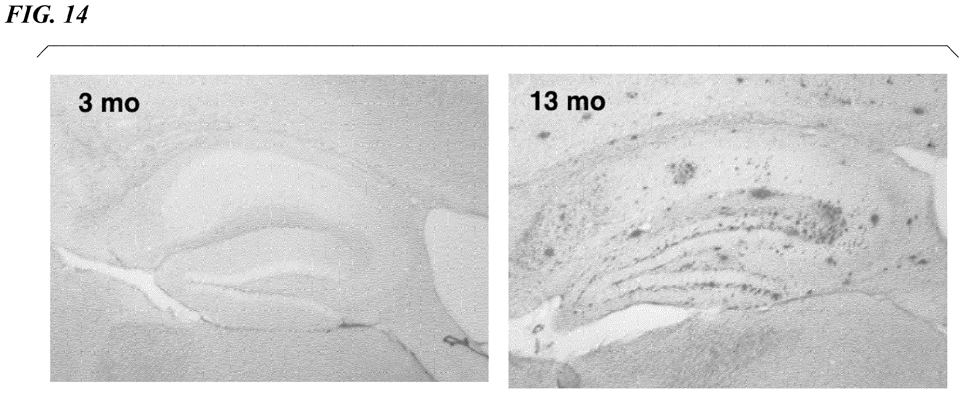

A brain tissue of the 3-month-old or 13-month-old human familial mutant APP gene-introduced mouse used in Experimental Example 2 was excised, the left hemisphere was fractionated and dissolved in 5 M guanidine hydrochloride, and then measurement of amyloid-β40 and amyloid-β42 concentrations in the mouse brain tissue was carried out using an Aβ ELISA kit (manufactured by FUJIFILM Wako Pure Chemical Corporation). In addition, the brain tissue was excised, the right hemisphere was fixed by immersion in 4% paraformaldehyde overnight to produce a sacrificial paraffin section. This section was subjected to immunohistochemical staining of amyloid-β, using an anti-amyloid-β monoclonal antibody (model "4G8", manufactured by Biolegend). FIG. 13 shows results of measurement of the amyloid-β40 and amyloid-β42 concentrations in the mouse brain tissue, and FIG. 14 shows results of immunohistochemical staining of amyloid-β in the mouse brain tissue. As shown in FIGS. 13 and 14, although amyloid-β was not accumulated in the brain tissue of the 3-month-old human familial mutant APP gene-introduced mouse, amyloid-β was accumulated in the brain tissue of the 13-month-old human familial mutant APP gene-introduced mouse.

From the results of Experimental Examples 2 and 3, it was clarified that amyloid-β was not detected in exosomes in the serum of the 3-month-old human familial mutant APP gene-introduced mouse in which amyloid-β was not accumulated in the brain tissue, but amyloid-β was detected in exosomes in the serum of the 13-month-old human familial mutant APP gene-introduced mouse in which amyloid-β was accumulated in the brain tissue. This result shows that an accumulation level of amyloid-β in the brain can be evaluated by detecting the amyloid-β bound to a brain-neuronal-cell-derived exosome in blood.

### [Comparative Example 1]

### (Detection of free amyloid-β in mouse serum)

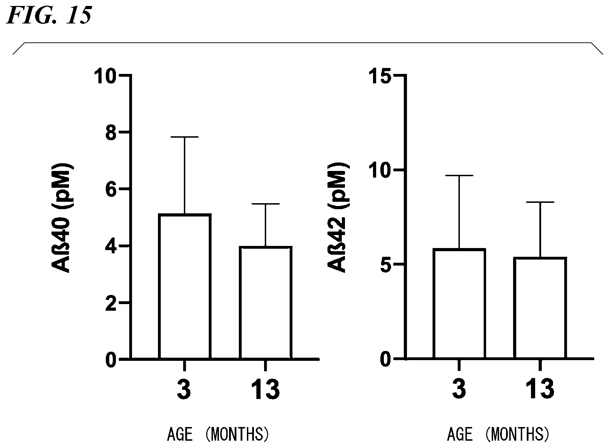

Measurement of free amyloid-β40 and amyloid-β42 concentrations in the 3-month-old or 13-month-old human familial mutant APP gene-introduced mouse serum used in Experimental Example 2 was carried out using an Aβ ELISA kit (manufactured by FUJIFILM Wako Pure Chemical Corporation). The results are shown in FIG. 15.

As shown in FIG. 15, free amyloid-β in serum changed in neither the 3-month-old human familial mutant APP gene-introduced mouse nor the 13-month-old human familial mutant APP gene-introduced mouse. This result shows that the accumulation level of amyloid-β in the brain cannot be evaluated by measuring free amyloid-β that is not bound to exosomes.

### Industrial Applicability

According to the present invention, it is possible to provide a technology of detecting amyloid-β bound to a brain-neuronal-cell-derived exosome rapidly and with high sensitivity and a technology of evaluating an accumulation level of amyloid-β in the brain.

### Reference Signs List

100: Complex
110: Brain-neuronal-cell-derived exosome
111: Amyloid-β
112: Biomarker
120: Amyloid-β-specific binding substance
121, 151: Nucleic acid fragment
130: Invasion probe
150: Biomarker-specific binding substance on surface of exosome
200, 500: Fluid device
210: Substrate
220: Lid member
221: Convex portion
222: Introduction port
223: Discharge port
230: Flow path
240: Well array
241: Well
242: Sealed well (micro-compartment)
L210: Reagent solution
L220: Sealing liquid
242R: Well in which signal is detected
510: Wall member
810: Flap probe
811: Flap site (nucleic acid fragment)
821: Second flap site (nucleic acid fragment)
820, 820': Nucleic acid fragment
F: Fluorescent substance
Q: Quenching substance

## Claims

1. A method for detecting amyloid-β bound to a brain-neuronal-cell-derived exosome in blood, the method comprising:
a step of binding amyloid-β bound to the brain-neuronal-cell-derived exosome in blood to an amyloid-β-specific binding substance that specifically binds to amyloid-β, labeled with a nucleic acid fragment; and
a step of detecting the amyloid-β-specific binding substance.

2. The detection method according to Claim 1, further comprising:
a step of detecting a biomarker present on a surface of the brain-neuronal-cell-derived exosome.

3. A method for detecting amyloid-β bound to a brain-neuronal-cell-derived exosome in blood, the method comprising:
a step of binding a biomarker present on a surface of the exosome to a biomarker-specific binding substance on the surface of the exosome, which specifically binds to the biomarker present on the surface of the exosome, labeled with a nucleic acid fragment;
a step of detecting amyloid-β bound to the brain-neuronal-cell-derived exosome in blood; and
a step of detecting the biomarker-specific binding substance on the surface of the exosome.

4. The detection method according to any one of Claims 1 to 3,
wherein the amyloid-β is bound to a biomarker present on a surface of the brain-neuronal-cell-derived exosome.

5. The detection method according to any one of Claims 2 to 4,
wherein the step of detecting the biomarker or the amyloid-β is carried out by capturing the brain-neuronal-cell-derived exosome using a substance that specifically binds to the biomarker or the amyloid-β.

6. The detection method according to any one of Claims 1 to 5,
wherein the amyloid-β-specific binding substance is an antibody or antibody fragment that specifically binds to amyloid-β.

7. The detection method according to any one of Claims 2 to 6,
wherein the biomarker is ganglioside GM1.

8. The detection method according to any one of Claims 1 to 7,
wherein the step of detecting the amyloid-β-specific binding substance is carried out by an invasive cleavage assay.

9. A kit for detection of amyloid-β bound to a brain-neuronal-cell-derived exosome in blood, the kit comprising:
an amyloid-β-specific binding substance that specifically binds to amyloid-β, labeled with a nucleic acid fragment.

10. The kit according to Claim 9, further comprising:
a substance that detects a biomarker present on a surface of the brain-neuronal-cell-derived exosome.

11. The kit according to Claim 10,
wherein the substance that detects a biomarker present on the surface of the brain-neuronal-cell-derived exosome is a substance that specifically binds to the biomarker present on the surface of the brain-neuronal-cell-derived exosome.

12. A kit for detection of amyloid-β bound to a brain-neuronal-cell-derived exosome in blood, comprising:
a biomarker-specific binding substance on a surface of a brain-neuronal-cell-derived exosome, which specifically binds to a biomarker present on the surface of the exosome, labeled with a nucleic acid fragment; and
an amyloids-specific binding substance that specifically binds to amyloid-β.

13. The kit according to any one of Claims 9 to 12,
wherein the amyloid-β-specific binding substance is an antibody or antibody fragment that specifically binds to amyloid-β.

14. The kit according to any one of Claims 10 to 13,
wherein the biomarker is ganglioside GM1.

15. A method for evaluating an accumulation level of amyloid-β in a brain, the method comprising:
a step of detecting amyloid-β bound to a brain-neuronal-cell-derived exosome in blood; and
a step of evaluating an accumulation level of amyloid-β in a brain based on the detected amount of the amyloid-β.

16. The evaluation method according to Claim 15,
wherein detection of the amyloid-β bound to the brain-neuronal-cell-derived exosome is carried out by the detection method according to any one of Claims 1 to 8.
